# DEMANDE DE BREVET EUROPEEN

(11) **EP 1 541 094 A1**
(43) Date de publication de la demande: **15.06.2005**
(21) Numéro de dépôt: 04292940.6
(22) Date de dépôt: 10.12.2004
(51) Int. Cl.: A61F 2/00

(54) **Dispositif d'aide à uriner des personnes de sexe féminin**

(30) Priorité: 12.12.2003 FR 0314565
(71) Demandeur: CIP Vehbi, 52000 Chaumont (FR)
(72) Inventeur: CIP Vehbi, 52000 Chaumont (FR)
(74) Mandataire: Honoré, Anne-Claire

(57) **Abrégé**

Dispositif d'aide pour le besoin d'uriner des femmes, formé par un tube (1) débouchant aux deux extrémités supérieure (2) et inférieure d'évacuation (3), présentant dans sa partie supérieure une portion de section ovale (4) dont l'extrémité (2) forme un orifice ovale (5) destiné à recevoir le méat urinaire, ladite extrémité (2) ayant une courbure dirigée vers l'intérieur du tube (1), adapté à l'anatomie de la vulve de l'utilisatrice, la section du tube (1) se réduisant progressivement en direction de l'extrémité opposée d'évacuation (3) remarquable en ce que la portion de section ovale (4) comporte au moins deux bourrelets de maintien (9) sur se face extérieure, situés à proximité de l'extrémité supérieure (2), de manière à assurer l'appui du tube (1) contre la vulve de l'utilisatrice par simple pression de ses doigts.

## Description

La présente invention concerne un dispositif d'aide à uriner des personnes de sexe féminin lorsqu'elles sont en position debout, assise ou allongée.

La prévention en urologie est un sujet d'actualité : de toutes les infections nosocomiales, les infections urinaires en constituent la fraction la plus importante, à savoir plus de 30%. Le plus grand nombre résulte des manipulations instrumentales. En effet, 70% de ces infections urinaires sont la conséquence d'un cathétérisme vésical. Les personnes âgées constituent une population à risque vis-à-vis des infections urinaires, qu'elles soient endogènes ou non.

Par ailleurs, comme chacun le sait, les toilettes publiques sont des lieux d'aisance où la propreté est la plupart du temps très douteuse. Les personnes de sexe féminin sont légitimement réticentes à ce que leur peau entre en contact à proximité de leurs parties intimes avec des objets (en particulier le sanitaire ou la lunette rabattable) potentiellement sales ou infectés. Cet aspect contribue à rendre l'acte d'uriner particulièrement contraignant pour celles qui n'osent toucher le sanitaire et à augmenter les chances d'infection pour celles qui s'y risquent.

Pour répondre à tous ces problèmes, on a déjà imaginé un dispositif d'aide pour uriner pour femmes, de forme globalement conique dont l'extrémité de plus grande section est d'une part inclinée par rapport à l'axe de cône et d'autre part, incurvée vers l'intérieur. C'est par exemple, le cas de la demande de brevet internationale WO 98/43563 où le dispositif est composé de deux parties, une première partie réalisée dans un matériau solide et imperméable et une seconde partie s'insérant dans la première, elle aussi de forme conique. La seconde partie est destinée à être jetée après utilisation. L'extrémité de grande section est adaptée à l'anatomie des femmes et destinée à recevoir le méat urinaire en son centre par maintien du tube en appui contre la vulve par l'utilisatrice. La première partie de forme conique comporte des moyens de préhension pour la main consistant d'une part, d'un côté, en une cavité apte à recevoir le pouce et d'autre part, de manière diamétralement opposée, en quatre cavités aptes à recevoir les quatre autres doigts. Ces cavités peuvent être réalisées de telle sorte que le dispositif puisse être maintenu en position que l'utilisatrice soit droitière ou gauchère.

Ce dispositif présente néanmoins des inconvénients que la présente invention vise à pallier. D'abord, ce type de maintien n'est pas aisé à mettre en oeuvre et parait critiquable en terme d'ergonomie. Les cavités recevant les doigts appartiennent au plan sagittal de la personne tenant le dispositif, c'est-à-dire le plan vertical orienté dans le sens antéro-postérieur sur la ligne médiane du corps. Ainsi, lorsque les doigts sont insérés dans leurs cavités respectives, la main et l'avant-bras sont en position de pronation, c'est-à-dire quand celle-ci se présente avec la paume en-dessous et le pouce à l'intérieur. Parallèlement à cela, le poignet est contraint dans une posture qui n'est pas anatomiquement favorable, se trouvant d'une part en déviation radiale, c'est-à-dire subissant une rotation interne, et d'autre part une extension forcée. Cette position forcée entraîne un confort très limité pendant l'utilisation du dispositif, et peut engendrer une fatigue importante lors d'applications longues et/ou répétées.

De même, ce type de dispositif présente l'inconvénient de ne pouvoir être appliqué contre la vulve de l'utilisatrice que par l'application d'une force manuelle. Or, notamment dans le domaine hospitalier, il est fréquent que le dispositif d'aide pour uriner doive être laissé en place pendant un temps plus ou moins long en fonction de l'utilité du dispositif, de la disponibilité des infirmières, de la capacité souvent réduite des personnes hospitalisées et/ou alitées... Ce type de dispositif est alors complètement inadapté pour ce type d'applications car les moyens de maintien en appui contre la vulve qu'il propose n'offrent en aucun cas la possibilité d'un recours à une application de force autre que manuelle afin de faciliter le recours au dispositif pendant une durée relativement longue.

La présente invention a pour objectif de remédier à tous ces inconvénients en proposant un dispositif d'aide pour uriner, aux personnes de sexe féminin, qu'elles soient debout, assises ou allongées, d'une part améliorant l'ergonomie lors de son utilisation et sa facilité d'emploi, et d'autre part offrant des moyens de maintien sur lesquels peut être appliquée une force manuelle ou non.

A cet effet, et conformément à l'invention, il est proposé un dispositif d'aide pour le besoin d'uriner des femmes formé par un tube débouchant aux deux extrémités supérieure et inférieure d'évacuation, présentant dans sa partie supérieure une portion de section ovale dont l'extrémité forme un orifice ovale destiné à recevoir le méat urinaire, ladite extrémité ayant une courbure dirigée vers l'intérieur du tube, adaptée à l'anatomie de la vulve de l'utilisatrice, la section du tube se réduisant progressivement en direction de l'extrémité opposée d'évacuation remarquable en ce que la portion de section ovale comporte au moins deux bourrelets de maintien sur sa face extérieure, situés à proximité de l'extrémité supérieure, de manière à assurer l'appui du tube contre la vulve de l'utilisatrice par simple pression de ses doigts.

On comprend bien que, contrairement aux dispositifs de l'art antérieur, les bourrelets de maintien au moment de l'utilisation, avantageusement situés symétriquement par rapport au plan sagittal, peuvent respectivement coopérer avec la face interne d'un unique doigt, par exemple avec l'index et le majeur, lors du maintien du dispositif contre la vulve par simple pression de ces doigts, de sorte que le poignet se trouve parfaitement dans le prolongement de l'avant-bras, diminuant d'autant la fatigue inhérente à des applications prolongées et/ou répétées. De plus, ce type de maintien en forme de bourrelets offre la possibilité, lors de certaines applications, d'utiliser le dispositif avec un dispositif externe maintenant le dispositif d'aide pour uriner en place sans application manuelle et pendant des périodes prolongées.

D'autres caractéristiques et avantages ressortiront encore mieux de la description de toutes les variantes qui vont être données d'un exemple non limitatif d'un dispositif d'aide pour uriner selon la présente invention, en référence aux dessins annexés sur lesquels :
- la figure 1 représente une vue de face du dispositif d'aide pour uriner conforme à l'invention,
- la figure 2 représente une vue de droite du dispositif de la figure 1,
- la figure 3 représente une vue en coupe du dispositif de la figure 1,
- la figure 4 représente le dispositif de la figure 1 en cours d'utilisation lorsque l'utilisatrice est debout ou assise,
- la figure 5 représente le dispositif de la figure 1 en cours d'utilisation lorsque l'utilisatrice est allongée,
- la figure 6 représente le dispositif de la figure 1 adapté pour recueillir l'urine dans un récipient lorsque l'utilisatrice est debout ou assise,
- la figure 7 représente le dispositif de la figure 1 maintenu contre la vulve de l'utilisatrice par un harnais.

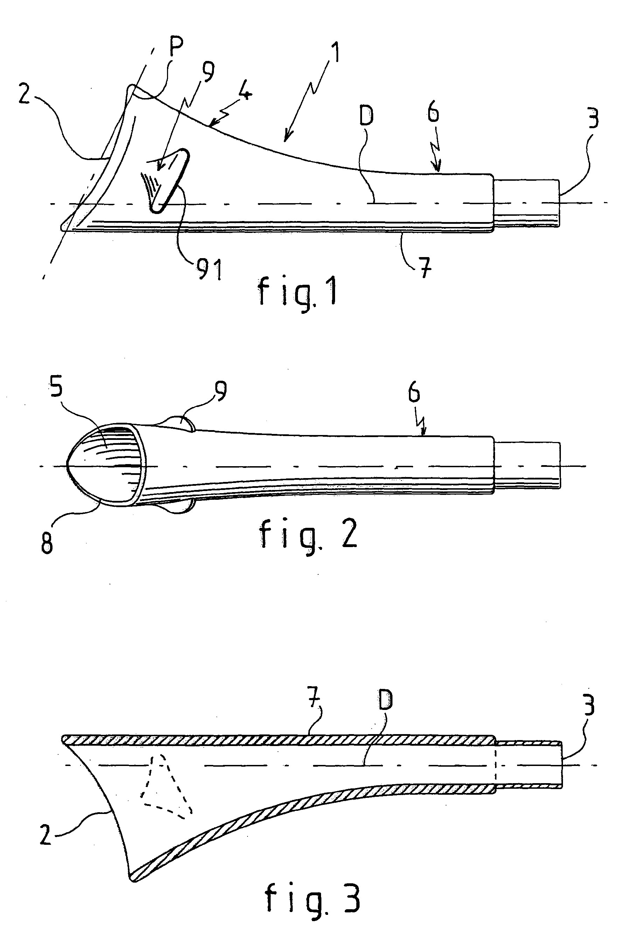

En référence aux figures 1, 2 et 3, le dispositif d'aide pour uriner est formé par un tube 1 débouchant aux deux extrémités supérieure et inférieure 2, 3. Le tube 1 présente dans sa partie supérieure, c'est-à-dire à proximité de l'extrémité 2, une portion de section ovale 4 dont l'extrémité 2 forme un orifice ovale 5 destiné à recevoir le méat urinaire lorsque l'extrémité 2 est appliquée contre la vulve de l'utilisatrice. L'orifice ovale 5 possède globalement un grand axe compris dans le plan sagittal de l'utilisatrice lorsque celle-ci utilise le dispositif. Afin de s'adapter à l'anatomie de l'utilisatrice, l'extrémité 2 possède une courbure dirigée vers l'intérieur du tube 1. La section du tube 1 se réduit progressivement en direction de l'extrémité opposée d'évacuation 3. Le tube 1 est d'épaisseur constante (figure 3), sauf à proximité de l'extrémité 3 comme il le sera décrit plus loin, et comporte dans sa partie inférieure une portion cylindrique 6 d'axe de révolution D. Enfin, la paroi du tube 1 est agencée spatialement de telle manière qu'elle ne possède qu'une fibre neutre 7 qui soit rectiligne, toutes les autres étant courbées. Elle est de surcroît parallèle à l'axe D et inclue l'un des deux points de courbure maximale de toute section pratiquée dans le tube 1. Elle coïncide avec une unique génératrice de la partie cylindrique 6. Néanmoins, il va de soi que le dispositif pourrait comporter un corps quelconque dès lors que celui-ci possède au moins une portion de section ovale se réduisant en direction de l'extrémité opposée à celle recevant le méat urinaire sans pour autant que cette variante ne sorte du cadre de l'invention.

Le contour 8 de l'orifice 5 situé à l'extrémité supérieure 2 du tube 1 possède un plan moyen P, le contour 8 s'étendant de part et d'autre du plan P en raison de la courbure déjà évoquée. L'angle que forme la fibre neutre rectiligne 7 par rapport au plan P dans le plan de la figure 1 qui est parallèle au plan sagittal lorsque l'utilisatrice utilise le dispositif est repéré α (figure 1). La valeur de α est en pratique comprise entre 50 degrés et 90 degrés. Néanmoins, une solution où α n'appartiendrait pas à cet intervalle ne sortirait en aucun cas du cadre de l'invention.

Dans un autre aspect de l'invention, la portion de section ovale 4 comporte deux bourrelets de maintien 9 sur sa face extérieure et situés à proximité de l'extrémité supérieure 2. Ces bourrelets 9 sont disposés symétriquement par rapport au plan sagittal de l'utilisatrice lorsque le dispositif est en appui contre la vulve. On pourrait néanmoins envisager une répartition des bourrelets telle qu'ils appartiendraient simultanément globalement au plan sagittal de l'utilisatrice. Enfin, toute solution où le nombre de bourrelets 9 est supérieur ou égal à deux et dont la répartition est quelconque ne saurait sortir du cadre de l'invention.

Avantageusement, chaque bourrelet 9 présente une surface d'appui 91 globalement parallèle au plan moyen P du contour 8 de manière à ce que l'utilisatrice puisse assurer l'appui du tube 1 contre sa vulve par simple pression de ses doigts sur les surfaces 91. En pratique, un seul doigt vient appuyer sur la surface 91 de chaque bourrelet 9. Les deux doigts couramment utilisés sont l'index et le majeur. Néanmoins, les surfaces 91 peuvent être de dimensions telles que chacune peut coopérer avec au moins deux doigts sans sortir du cadre de l'invention.

Le contour 8 de l'orifice ovale 5 a une forme arrondie et présente une élasticité permettant ainsi un contact répété entre le dispositif et la peau de l'utilisatrice sans pour autant ni la blesser, ni l'irriter.

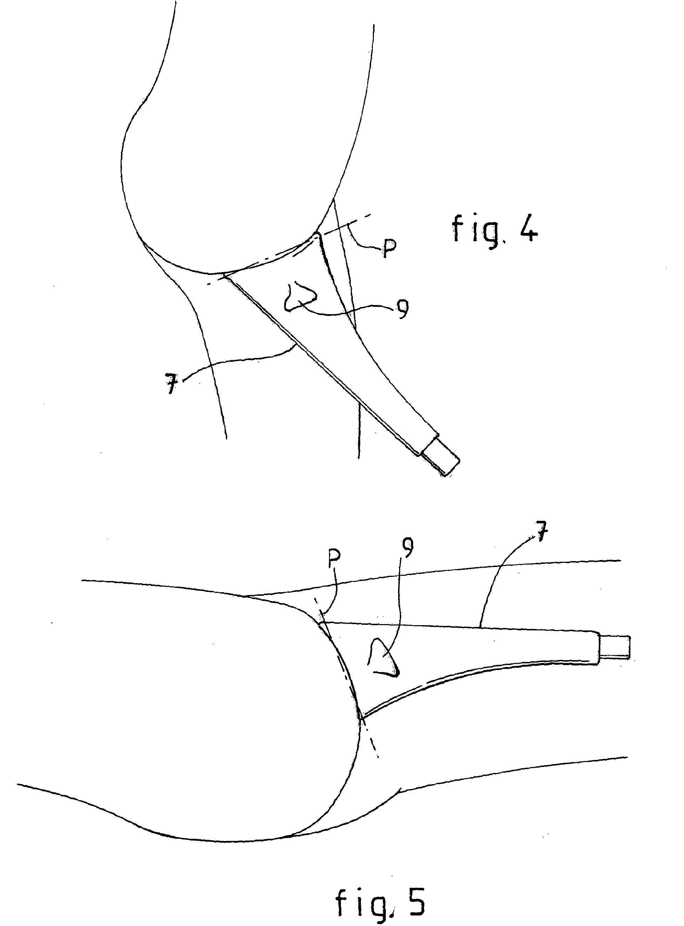

En référence aux figures 4 et 5, le dispositif peut être utilisé debout ou en position assise (figure 4), ou bien encore en position allongée (figure 5). Dans les deux cas de figures, le plan moyen P est globalement perpendiculaire à l'axe tête-pied de l'utilisatrice et la fibre neutre rectiligne 7 appartient globalement à son plan sagittal. Tandis que lorsque l'utilisatrice est allongée, la fibre neutre rectiligne 7 est globalement colinéaire avec l'axe tête-pied de celle-ci, en position debout ou assise la fibre 7 forme un angle complémentaire de l'angle α avec ledit axe. Ainsi, le passage d'une configuration à l'autre est obtenu par simple rotation du dispositif de 180 degrés d'axe l'axe tête-pied de l'utilisatrice.

En pratique, que le tronc de l'utilisatrice soit horizontal (position allongée) ou vertical (position debout ou assise), le positionnement du plan moyen P est identique par rapport audit tronc, à savoir perpendiculaire à l'axe tête-pied de l'utilisatrice. Les surfaces 91 des bourrelets 9 étant globalement parallèles au plan P, le positionnement des ces surfaces et leur inclinaison sont identiques quelle que soit la configuration. Ainsi, l'accès de la main aux surfaces 91 est indépendant de la configuration (debout, assis, allongé). De plus, ces accès est particulièrement aisé. Lorsque l'index et le majeur coopèrent avec le bourrelet de maintien 9 correspondant, la main se trouve dans le prolongement du poignet qui ne subit alors ni déviation radiale ni extension forcée. Ces aspect est fondamental en terme de fatigue, de confort, d'ergonomie lors d'utilisations longues et/ou répétées, car il contribue à sérieusement diminuer les douleurs et fatigues inhérentes à ces utilisations.

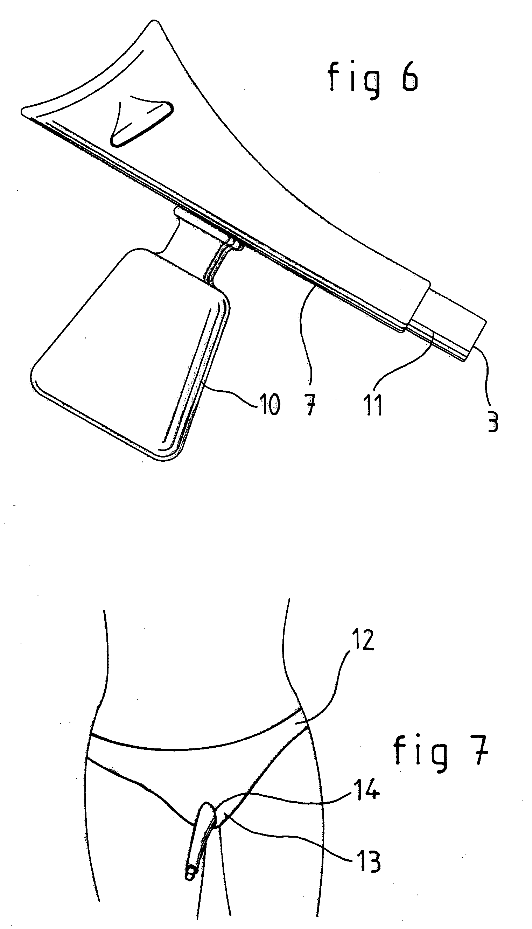

Dans certaines applications, notamment hospitalières, le dispositif sert à collecter l'urine pour une utilisation ultérieure. A cet effet, et en référence à la figure 6, le dispositif peut comporter un opercule frangible disposé le long de la fibre rectiligne 7 afin de pouvoir faire communiquer l'intérieur du tube 1 (où s'écoule l'urine) avec l'extérieur. L'opercule est une portion détachable en tout ou partie par fracture dont le contour de rupture est une zone d'épaisseur réduite du matériau constitutif du tube 1. Plus précisément, la zone d'épaisseur réduite comporte une rainure formée dans une des surfaces intérieure ou extérieure du tube 1. Le contour de rupture est conçu de telle manière que le goulot d'un récipient de collecte 10 puisse être introduit de manière étanche dans l'espace libéré par l'opercule fracturé. Toute solution connue de *l'Homme du Métier* pour y parvenir pourra être mise en oeuvre sans sortir du cadre de l'invention. Ce mode de collecte sera utilisé lorsque l'utilisatrice est debout ou assise.

Par ailleurs, le tube 1 comporte à proximité de l'extrémité inférieure d'évacuation 3 du tube 1, une réduction de l'épaisseur de sa paroi, sans création de discontinuité de la surface intérieure, suivant une longueur sensiblement égale au double du diamètre intérieur du tube 1 à l'extrémité d'évacuation 3, de manière à créer un col 11. Une durite (non représentée) peut être emmanchée à force sur le col 11 afin de raccorder l'extrémité d'évacuation 3 à un récipient de collecte d'urine situé à distance du dispositif, sans que l'épaisseur de ladite durite ne dépasse de la surface extérieure du tube 1 afin de diminuer les risques de désolidarisation involontaire de la durite. Ce mode de collecte de l'urine sera utilisé lorsque l'utilisatrice est alitée.

Lorsqu'il est nécessaire de maintenir le dispositif en appui contre la vulve pendant une longue période, le maintien peut être obtenu à l'aide d'un harnais 12 enserrant la taille de l'utilisatrice (figure 7) et recouvrant la vulve et les fesses. Ce harnais 12 peut être obtenu par assemblage de sangles, de tissus, de plastique ou de tout autre matériau ou accessoire, et ce conformément à tout mode de réalisation connu de *l'Homme du Métier*. De plus, le harnais 12 peut comporter un moyen de réglage de la pression exercée sur la vulve par la partie 13 recouvrant la vulve, cette partie 13 étant évidée selon une forme ovale qui crée un évidement 14, de manière à ce que le dispositif d'aide pour uriner puisse y être introduit par insertion de l'extrémité d'évacuation 3 jusqu'à ce que le contour de l'évidement 14 entre en contact avec les surfaces 91 des bourrelets de maintien 9. Le contour de l'évidement 14 doit présenter une rigidité suffisante pour pouvoir appliquer une force sur les surfaces 91 sans que ledit contour ne se déforme. Il va de soi que le dispositif doit être enfilé dans l'évidement 14 du harnais 12 avant que ce dernier ne soit fixé sur l'utilisatrice. Le moyen de réglage de la pression, consistant par exemple en un système classique de serrage pour sangle, permet d'ajuster la pression que l'extrémité supérieure 2 du tube 1 exerce sur la vulve de l'utilisatrice. Bien entendu, tout autre moyen de serrage équivalent peut convenir.

Ainsi, le dispositif conforme à l'invention est complètement adapté pour des utilisations longues et/ou répétées car les bourrelets de maintien 9 permettant l'appui contre la vulve de l'utilisatrice peuvent recevoir une force d'appui au niveau des surfaces 91 autre que manuelle, en particulier par coopération avec un harnais conçu à cet effet.

Le dispositif (à usage unique ou non) peut être obtenu dans tout matériau étanche et biodégradable. Par exemple, il peut être réalisé en carton ou en papier mâché, recouvert intérieurement d'une couche aluminium ou d'un plastifiant pour l'imperméabilité. Il peut également être obtenu par injection dans un moule d'une matière plastique.

Le dispositif, vendu sous sachet étanche, préalablement stérilisé, peut, par exemple, être obtenu par pliage d'une feuille d'un des matériaux précités. Cette feuille possède alors une forme particulière qui, par pliage, rabattement, puis collage donne le dispositif tel qu'utilisable. Le pliage s'effectue au niveau de lignes prédéterminées.

Enfin, il va de soi que diverses modifications peuvent être apportées par *l'Homme du Métier* au dispositif qui vient d'être décrit uniquement à titre d'exemple non limitatif, sans sortir du cadre de l'invention.

## Revendications

1. Dispositif d'aide pour le besoin d'uriner des femmes, formé par un tube (1) débouchant aux deux extrémités supérieure (2) et inférieure d'évacuation (3), présentant dans sa partie supérieure une portion de section ovale (4) dont l'extrémité (2) forme un orifice ovale (5) destiné à recevoir le méat urinaire, ladite extrémité (2) ayant une courbure dirigée vers l'intérieur du tube (1), adaptée à l'anatomie de la vulve de l'utilisatrice, la section du tube (1) se réduisant progressivement en direction de l'extrémité opposée d'évacuation (3) **caractérisé en ce que** la portion de section ovale (4) comporte au moins deux bourrelets de maintien (9) sur sa face extérieure, situés à proximité de l'extrémité supérieure (2), de manière à assurer l'appui du tube (1) contre la vulve de l'utilisatrice par simple pression de ses doigts.

2. Dispositif selon la revendication précédente **caractérisé en ce que** les bourrelets de maintien (9) sont disposés symétriquement par rapport au plan sagittal de l'utilisatrice lorsque ledit dispositif est en appui contre sa vulve.

3. Dispositif selon l'une quelconque des revendications précédentes **caractérisé en ce qu'**il comporte dans sa partie inférieure une portion cylindrique (6).

4. Dispositif selon l'une quelconque des revendications précédentes **caractérisé en ce que** la paroi du tube (1) possède une fibre neutre rectiligne (7), ladite ligne (5) incluant l'un des deux points de courbure maximale de toute section pratiquée dans le tube (1).

5. Dispositif selon la revendication précédente **caractérisé en ce que** le contour (8) de l'orifice ovale (5) possède un plan moyen (P), le contour (8) s'étendant de part et d'autre dudit plan (P) qui est incliné d'un angle α par rapport à la fibre neutre (7) de valeur comprise entre 50 degrés et 90 degrés.

6. Dispositif selon la revendication 5 **caractérisé en ce que** chaque bourrelet de maintien (9) comporte une surface d'appui (91) globalement parallèle au plan moyen (P) de sorte que l'utilisatrice assure l'appui du tube (1) contre sa vulve par pression des doigts sur les surfaces (91).

7. Dispositif selon l'une quelconque des revendications précédentes **caractérisé en ce qu'**il comporte un opercule frangible afin de faire communiquer l'intérieur du tube (1) avec l'extérieur, l'opercule étant une portion détachable en tout ou partie par fracture dont le contour de rupture est une zone d'épaisseur réduite du matériau constitutif du tube (1), ledit contour de rupture étant conçu de telle manière que le goulot d'un récipient de collecte (10) puisse être introduit de manière étanche dans l'espace libéré par l'opercule fracturé.

8. Dispositif selon l'une quelconque des revendications précédentes **caractérisé en ce que** le tube (1) comporte à proximité de l'extrémité d'évacuation (3) une diminution de l'épaisseur de sa paroi, sans création de discontinuité de la surface intérieure, de manière à former un col (11) apte à coopérer par emmanchement à force avec une durite servant à raccorder l'extrémité d'évacuation (3) à un récipient de collecte d'urine.

9. Dispositif selon l'une quelconque des revendications précédentes **caractérisé en ce qu'**il est obtenu dans tout matériau étanche et biodégradable.

10. Dispositif selon la revendication précédente **caractérisé en ce qu'**il est réalisé en carton ou en papier mâché, recouvert intérieurement d'une couche d'aluminium ou d'un plastifiant, ou obtenu par injection dans un moule d'une matière plastique.
